# EUROPEAN PATENT APPLICATION

(11) **EP 4 722 353 A1**
(43) Date of publication of application: **08.04.2026**
(21) Application number: 23939277.2
(22) Date of filing: 08.11.2023
(51) Int. Cl.: C12N 7/01, C12N 15/869, C12N 15/55, C12N 15/113, A61K 35/763, A61K 48/00, A61K 38/50, A61P 35/00, C12R 1/93

(54) **RECOMBINANT HERPES SIMPLEX VIRUS, AND PREPARATION METHOD THEREFOR AND USE THEREOF**

(30) Priority: 30.05.2023 CN 202310618884
(71) Applicant: Zhongyi (Suzhou) Biopharmaceutical Co., Ltd., Suzhou, Jiangsu 215127 (CN)
(72) Inventor: LIU, Fusheng, Beijing 101520 (CN)
(74) Representative: Valet Patent Services Limited
(86) International application number: PCT/CN2023/130345
(87) International publication number: WO 2024/244306

(57) **Abstract**

The present invention relates to the technical field of genetic engineering. Disclosed are a recombinant herpes simplex virus, and a preparation method therefor and the use thereof. The recombinant herpes simplex virus comprises a vector and a nucleotide sequence for reducing the expression level of a DPD gene, wherein the vector is a herpes simplex virus which lacks an ICP47 coding gene and contains a CD gene, and the insertion site of the nucleotide sequence for reducing the expression level of the DPD gene is the position on the vector at which the ICP47 coding gene is deleted. The recombinant herpes simplex virus inserts shRNA interfering with DPD into HSV-CD, thereby improving the killing ability of CD/5-FC on glioma cells while interfering with the metabolism of the glioma cells.

## Description

### TECHNICAL FIELD

The invention relates to the technical field of genetic engineering, and specially to a recombinant herpes simplex virus, a preparation method therefor, and use thereof.

### BACKGROUND

Oncolytic viruses have been used for cancer treatment in various preclinical and clinical studies. The oncolytic viruses acquire a tumor-killing effect by deleting specific genes on the viral genome to give the oncolytic viruses an ability of selective replicatication and amplification in tumor cells. At the same time, the modified oncolytic viruses cannot replicate in normal cells, and thus have high safety. Herpes simplex virus I (HSV-1) is currently one of the most widely used oncolytic viruses in clinic.

At present, genetical engineering means are used to modify wild-type (WT) HSV-1 by deleting specific gene(s), and then the modified virus are used as gene expression vector in human gene therapy research so that a therapeutic gene is expressed to cooperate with oncolytic virus therapy during oncolysis. On the basis of the modified WT HSV-1, two expression frames are inserted, one of which can express cytosine deaminase (CD) in tumor cells where the CD can transform an exogenous non-toxic 5-FC (a prodrug 5-fluorocytosine) to a chemotherapeutic drug 5-FU (5-fluorouracil) to interfere with the DNA synthesis in the tumor cells, and the other of which inserts a TSPO gene-interfering shRNA to reduce the expression. After three rounds of modifications, a HSV-CD-shTSPO oncolytic virus is constructed.

The above-described recombinant herpes simplex virus can effectively reduce the growth rate of glioma and provide sufficient time for HSV-1 oncolytic virus to kill glioma cells, which improves the killing efficiency of glioma, and has less side effect after use. However, although the TSPO gene-interfering shRNA inserted in this technology can function to interfere with the metabolism of glioma cells, it is not directly associated with the CD gene inserted in the virus and cannot directly enhance the killing function of CD/5-FC Function.

### SUMMARY

To the above problems, the first object of the present invention is to provide a recombinant herpes simplex virus, which inserts a DPD-interfering shRNA into an HSV-CD, enhancing the killing ability of CD/5-FC on glioma cells while interfering with the glioma cell metabolism.

The second object of the present invention is to provide a method for preparing the recombinant herpes simplex virus.

The third object of the present invention is to provide use of the recombinant herpes simplex virus in the preparation of a drug for treating glioma.

The first technical solution adopted in the present invention is: a recombinant herpes simplex virus comprising a vector and a nucleotide sequence for reducing the expression level of DPD gene;
wherein the vector is a herpes simplex virus in which an ICP47-encoding gene is deleted and a CD gene is included, wherein the insersion site of the nucleotide sequence for reducing the expression level of DPD gene is a site on the vector where the ICP47-encoding gene is deleted; and
the nucleotide sequence for reducing the expression level of DPD gene is as shown in SEQ ID NO: 1, SEQ ID NO: 2, SEQ ID NO: 3, or SEQ ID NO: 4.

Preferably, the insersion site of the CD gene in the vector is a site where the ICP34.5-encoding gene is deleted.

Preferably, the DPD gene has a nucleotide sequence as shown in SEQ ID NO: 5.

Preferably, the CD gene has a nucleotide sequence as shown in SEQ ID NO: 6.

Preferably, the vector is obtained by means of:
knocking out the ICP34.5-encoding gene in the herpes simplex virus, and inserting the CD gene into the site where the ICP34.5-encoding gene is knocked out, to afford a CD gene-containing herpes simplex virus; and
knocking out the ICP47-encoding gene in the CD gene-containing herpes simplex virus, to afford the vector.

The second technical solution adopted by the present invention is: a method for preparing the recombinant herpes simplex virus as described in the first technical solution, comprising the steps of:
knocking out the ICP47-encoding gene in the CD gene-containing herpes simplex virus to afford a vector; and inserting a nucleotide sequence for reducing the expression level of DPD gene into the vector; wherein an insertion site of the nucleotide sequence for reducing the expression level of DPD gene is a site on the vector where the ICP47-encoding gene is knocked out; and
wherein the nucleotide sequence for reducing the expression level of DPD gene is as shown in SEQ ID NO: 1, SEQ ID NO: 2, SEQ ID NO: 3, or SEQ ID NO: 4.

Preferably, the CD gene-containing herpes simplex virus is obtained by means of: knocking out the ICP34.5-encoding gene in the herpes simplex virus, and inserting the CD gene into the site where the ICP34.5-encoding gene is knocked out, to afford the CD gene-containing herpes simplex virus.

Preferably, the method for preparing the recombinant herpes simplex virus further comprises:
synthesizing the CD gene, and cloning the CD gene into the pSP72ΔICP34.5CMVWPRE vector using Hind III and Xho I to afford pSP72ΔICP34.5CMVCDWPRE; co-transfecting BHK cells with pSP72ΔICP34.5CMVCDWPRE and HSV-1ΔICP34.5CMVEGFP, wherein virus spots that do not express a green fluorescence indicate that the gene EGFP in the original virus is replaced by CD gene recombination; purifying and growing the HSV-1ΔICP34.5CMVCDWPRE virus vector, and extracting a full-length DNA of the vector, to afford the CD gene-containing herpes simplex virus.

The third technical solution adopted by the present invention is: use of the recombinant herpes simplex virus according to the first technical solution or the recombinant herpes simplex virus prepared by the method as described in the second technical solution in the preparation of a drug for the treatment of glioma.

The above technical solutions have the following beneficial effects:
(1) In the present invention, the DPD-interfering shRNA is inserted into the HSV-CD to construct the HSV-CD-shDPD oncolytic virus *(i.e.,* the recombinant herpes simplex virus), and the recombinant herpes simplex virus effectively reduces the metabolism, invasion and migration of glioma, while improving the utilization rate of the inserted CD gene to the prodrug 5-FC, and increasing the half-life of 5-FU transformed by the CD gene and the chemotherapy efficiency in glioma cells. Namely, it simultaneously improves the utilization rate of exogenous drugs, and increases the half-life of chemotherapeutic drugs and the chemotherapy efficiency in glioma cells.

### BRIEF DESCRIPTOIN OF DRAWINGS

FIG. 1 is a graph showing the relationship between different pathological grades of glioma and the expression of DPD;
FIG. 2 is a graph showing the relationship between the DPD expression and the prognosis in primary gliomas;
FIG. 3 is a graph showing the relationship between the DPD expression and the prognosis in recurrent glioma;
FIG. 4 is a schematic diagram of the DPD expression in glioma pathological tissue as detected by immunohistochemistry, where the "Low grade" is a low grade and the "High grade" is a high grade;
FIG. 5 is a diagram of the interference efficiency of different DPD-interfering shRNAs;
FIG. 6 is a graph showing the effect of interfering with the DPD expression in glioma cell U251 to inhibit the ability of the cell invasion and migration;
FIG. 7 is a diagram showing the effect of interfering with the DPD expression in glioma cells and enhancing the ability of 5-FU to kill cells; and
FIG. 8 is a diagram showing the comparison of the utilization rates of sh-DPD-1#, sh-DPD-3# and sh-TSPO to the 5-FC.

### BRIEF DESCRIPTION

The present invention will be further described below by specific examples. It should be pointed out that those of ordinary skill in the art can made several modifications and improvements without departing from the principles of the present invention, which should also be deemed within the protection scope of the presentinvention.

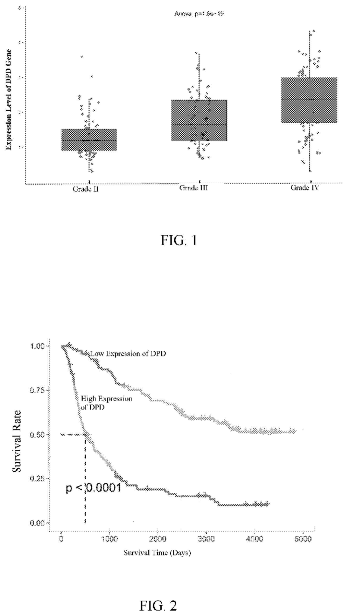

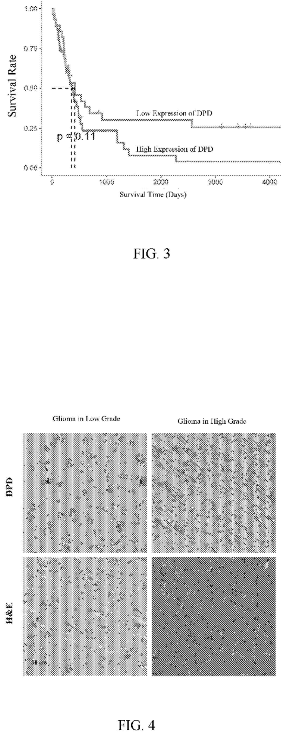

DPD (dihydropyrimidine dehydrogenase) is an initiator enzyme and rate-limiting enzyme of 5-FU (5-fluorouracil) metabolism *in vivo,* which can transform 5-FU in cells into non-toxic dihydrofluorouracil, which is an inhibitory enzyme of CD (cytosine deaminase)/5-FC system. DPD gene (DPYD) not only participates in the metabolic process but also participates in the tumor EMT process. The present inventors conducted a bioinformatics analysis based on the Chinese Glioma Genome Atlas Project (CGGA) database to analyze the relationship between the expression of DPD (dihydropyrimidine dehydrogenase) and the prognosis of glioma. It is found that, as shown in FIG. 1, the higher the pathological grade of glioma (grade II, III, and up to IV), the higher the expression of DPD; and as shown in FIG. 2 and FIG. 3, in the primary glioma and the recurrent glioma, the expression of DPD is high, and the prognosis of glioma is poor, that is, the DPD (dihydropyrimidine dehydrogenase) is generally highly expressed in the pathological tissues of glioma, and the higher the pathological grade, the higher the expression of DPD. By interfering with the DPD expression in glioma cells *in vitro,* it not only inhibits the invasion and migration of glioma cells, but also enhances the ability of 5-FU to kill glioma cells.

According to the above results of bioinformatics analysis, the expression of DPD in low-grade (II) and high-grade (III or IV) pathological tissues was analyzed by immunohistochemistry, and it was found that the expression of DPD in the high grade was higher than that in in low-grade, as shown in FIG. 4, which further verifies the above analysis results.

Based on the above analysis, in an aspect, the present invention provides a recombinant herpes simplex virus comprising a vector and a nucleotide sequence for reducing the expression level of DPD gene *(i.e.,* a DPD-interfering shRNA); wherein the vector is a herpes simplex virus in which an ICP47-encoding gene is deleted and a CD gene is included, and an insertion site of the nucleotide sequence for reducing the expression level of DPD gene is a site on the vector where the ICP47-encoding gene is deleted;
wherein the nucleotide sequence for reducing the expression level of DPD gene is as shown in SEQ ID NO: 1, SEQ ID NO: 2, SEQ ID NO: 3, or SEQ ID NO: 4; preferably a nucleotide sequence as shown in SEQ ID NO: 1 or SEQ ID NO: 3; and in the herpes simplex virus in which the ICP47-encoding gene is deleted and the CD gene is included, an insertion site of the CD gene is a site where the ICP34.5-encoding gene is deleted.

In the present invention, both the ICP34.5-encoding gene and the ICP47-encoding gene are well known to those skilled in the art, and can also be found by accessing relevant databases. For example, the relevant nucleotide sequences can be found by accessing the GenBank database. These are all conventional technical means for those skilled in the art, and are not reiterated in the present invention.

The DPD gene has a nucleotide sequence as shown in SEQ ID NO: 5.

The CD gene has a nucleotide sequence as shown in SEQ ID NO: 6.

The prodrug 5-fluorocytosine (5-FC) has no effect on cells. The 5-FU transformed by the deamination of 5-FC has a killing effect on both normal cells and tumor cells. This process can be catalyzed by cytosine deaminase (CD), and such deaminase is however lacking in the human body. Cytosine deaminase (an expression product of CD gene) can transform the initially non-toxic prodrug 5-FC into the cytotoxic chemotherapeutic drug 5-FU.

In the present invention, the type of the herpes simplex virus is not particularly limited, which can be any routine choice in the art. However, in order to better achieve the purpose of stably expressing cytokines, the herpes simplex virus is preferably Type I herpes simplex virus. Also, the source of the herpes simplex virus is not particularly limited in the present invention, which can be conventionally commercially available, or can be obtained by self-isolation in the laboratory.

In another aspect, the present invention also provides a method for preparing a recombinant herpes simplex virus, comprising: knocking out an ICP47-encoding gene in a CD gene-containing herpes simplex virus to afford a vector; and inserting a nucleotide sequence for reducing the expression level of DPD gene into the vector; wherein an insertion site of the nucleotide sequence for reducing the expression level of DPD gene is a site on the vector where the ICP47-encoding gene is knocked out.

Among them, the CD gene-containing herpes simplex virus is obtained by means of: knocking out an ICP34.5-encoding gene in the herpes simplex virus, and inserting the CD gene into a site where the ICP34.5-encoding gene is knocked out, to afford the CD gene-containing herpes simplex virus.

The ICP34.5-encoding gene and the ICP47-encoding gene can be knocked out by various conventional methods in the art, which are not particularly limited in the pressent invention. For example, the knock-out can be performed by means of homologous recombination, by means of targeted knock-out; or by means of targeted knock-out with CRISPY. On the premise of understanding the purpose of the present invention and the viral vector as used in the present invention, those skilled in the art can realize the knockout of the above genes according to the conventional technical means mastered by them.

Also, the nucleotide sequence for reducing the expression level of DPD gene and the CD gene can be inserted by various conventional methods in the art. The insertion method can be directly inserting the target sequence into the selected insertion site. For example, the insersion can be performed by means of CRISPY, or can be performed by replacing a part of base sequences with homologous recombination to insert the target sequence.

The preparation examples for recombinant herpes simplex virus comprise, for example:
(1) synthesizing the CD gene, and cloning the CD gene into the pSP72ΔICP34.5CMVWPRE vector using Hind III and Xho I (inserting the ICP34.5 gene-containing HSV-1 DNA fragment into the BglII site of the plasmid pSP72, deleting the ICP34.5 gene-encoding fragment of NotI by restriction endonuclease digestion of NotI to afford the psP72ΔICP34.5; adding WPRE (Woodchuckhepatitispost-regulationElement) under the control of the IE promoter of human CMV virus to afford the pSP72ΔICP34.5CMVWPRE vector), to afford the pSP72ΔICP34.5CMVCDWPRE; co-transferring the BHK cells with the pSP72ΔICP34.5CMVCDWPRE and the HSV-1ΔICP34.5CMVEGFP (deleting the ICP34.5 gene from wild-type HSV-1 to afford the HSV-1ΔICP34.5; co-cloning it with the marker gene EGFP which expresses green fluorescent protein under the control of the IE promotor of human CMV virus to afford the HSV-1ΔICP34.5CMVEGFP), wherein virus spots which do not express green fluorescent plaques indicate that the gene EGFP in the original virus is replaced by the CD gene recombination, purifying and growing the HSV-1ΔICP34.5CMVCDWPRE virus vector, extracting the full-length DNA of the vector, to afford the CD gene-containing herpes simplex virus;
(2) artificially synthesizing the nucleotide sequence as shown in SEQ ID NO: 1, SEQ ID NO: 2, SEQ ID NO: 3, or SEQ ID NO: 4 (where Hunan Yada Fenghui New Materials Co., Ltd. was commissioned to perform the artificial synthesis) to afford the artificially synthesized first nucleotide sequence, second nucleotide sequence, third nucleotide sequence or the fourth nucleotide sequence; knocking out the ICP47 gene in the CD gene-containing herpes simplex virus, and inserting the above artificially synthesized first nucleotide sequence, second nucleotide sequence, third nucleotide sequence or fourth nucleotide sequence into the site where the ICP47 gene is knocked out, to afford a recombinant herpes simplex virus; and
(3) performing a digestion validation to validate the sequence of the recombinant herpes simplex virus (where Beijing Tianyi Huiyuan Biotechnology Co., Ltd. was commissioned to perform the sequencing validation), confirming that the reading frame of the recombinant herpes simplex virus was correct, and selecting viruses in which the nucleotide sequence for reducing the expression level of DPD gene was correctly inserted into the CD gene-containing herpes simplex virus as a recombinant herpes simplex virus.

The recombinant herpes simplex virus disclosed in the invention can be used in the preparation of a drug for treating glioma.

Hereinafter the present invention will be described in detail by way of examples.

### Example 1

### Preparation of the CD gene-containing herpes simplex virus:

The ICP34.5 gene in the wild-type HSV-1 virus (which has a gene sequence with GenBank number of NC_001806) was knocked out according to the method described in the Chinese Patent Application No. 2004100064921 (with the Authorization Announcement Number of CN1283803C), and the CD gene was inserted into the site on the HSV-1 virus where the ICP34.5 gene was knocked out, to afford the CD gene-containing herpes simplex virus *(i.e.,* HSV-CD).

### Example 2

The ICP47 gene in the CD gene-containing herpes simplex virus was knocked out (by the same knockout method as in Example 1), and the nucleotide sequence for reducing the expression level of DPD gene (DPYD-1#) as shown in SEQ ID NO: 1 (GCAATTTGCTACTGAGGTATT) was inserted the site wherein the ICP47 gene was knocked out. Then, a recombinant virus vector was constructed from the resultant virus and a plasmid vector. The recombinant virus vector successfully constructed was propagated on Vero cells at 37°C and 5% CO₂, and the multiplicity of infection was 0.1. After harvesting, cell debris was removed with a 0.65 µm filter, and the filtrate was purified by centrifugation at a high speed of 13000 rpm to afford a virus suspension with titer of 1×10⁸ pfu/mL, thereby obtaining the recombinant herpes simplex virus HSV-CD-shDPD-1# *(i.e.,* sh-DPD-1#), which was used for subsequent cell experiments.

### Example 3

The ICP47 gene in the CD gene-containing herpes simplex virus was knocked out (by the same knockout method as in Example 1), and a nucleotide sequence for reducing the expression level of DPD gene (DPYD-2#) as shown in SEQ ID NO: 2 (GCCGTATGATGTAGTGAATTT) was inserted to the site where the ICP47 gene was knocked. Then, a recombinant virus vector was constructed from the resultant virus and a plasmid vector. The recombinant virus vector successfully constructed was propagated on Vero cells at 37°C and 5% CO₂, and the multiplicity of infection was 0.1. After harvesting, cell debris was removed with a 0.65 µm filter, and the filtrate was purified by centrifugation at a high speed of 13000 rpm to afford a virus suspension with titer of 1×10⁸ pfu/mL, thereby obtaining the recombinant herpes simplex virus HSV-CD-shDPD-2# *(i.e.,* sh-DPD-2#), which was used for subsequent cell experiments.

### Example 4

The ICP47 gene in the CD gene-containing herpes simplex virus was knocked out (by the same knockout method as in Example 1), and a nucleotide sequence for reducing the expression level of DPD gene (DPYD-3#) as shown in SEQ ID NO: 3 (GCTATACAGTTTGATCCAGAA) was inserted to the site where the ICP47 gene was knocked. Then, a recombinant virus vector was constructed from the resultant virus and a plasmid vector. The recombinant virus vector successfully constructed was propagated on Vero cells at 37°C and 5% CO₂, and the multiplicity of infection was 0.1. After harvesting, cell debris was removed with a 0.65 µm filter, and the filtrate was purified by centrifugation at a high speed of 13000 rpm to afford a virus suspension with titer of 1×10⁸ pfu/mL, thereby obtaining the recombinant herpes simplex virus HSV-CD-shDPD-3# *(i.e.,* sh-DPD-3#), which was used for subsequent cell experiments.

### Example 5

The ICP47 gene in the CD gene-containing herpes simplex virus was knocked out (by the same knockout method as in Example 1), and a nucleotide sequence for reducing the expression level of DPD gene (DPYD-4#) as shown in SEQ ID NO: 4 (GCAAGTATAAGTTGTGCTTCC) was inserted to the site where the ICP47 gene was knocked. Then, a recombinant virus vector was constructed from the resultant virus and a plasmid vector. The recombinant virus vector successfully constructed was propagated on Vero cells at 37°C and 5% CO₂, and the multiplicity of infection was 0.1. After harvesting, cell debris was removed with a 0.65 µm filter, and the filtrate was purified by centrifugation at a high speed of 13000 rpm to afford a virus suspension with titer of 1×10⁸ pfu/mL, thereby obtaining the recombinant herpes simplex virus HSV-CD-shDPD-3# *(i.e.,* sh-DPD-3#), which was used for subsequent cell experiments.

### Comparative Example 1

A recombinant herpes simplex virus was constructed according to the method described in Example 2, except that only the ICP47 gene in the CD gene-containing herpes simplex virus was knocked out, and no nucleotide sequence for reducing the expression level of DPD gene was inserted, to afford the HSV-CD-shcon *(i.e.,* sh-con).

### Comparative example 2

A recombinant herpes simplex virus was constructed according to the method described in Example 2, except that the ICP47 gene in the CD gene-containing herpes simplex virus was knocked out, and then a nucleotide sequence for reducing the expression level of TSPO gene was inserted into the site where the ICP47 gene was knocked out, wherein the nucleotide sequence for reducing the expression level of TSPO gene is: TCACTCAACTACTGCGTATGTTCAAGAGACATACGCAGTAGTTGAGTGTTTTTT.

In the present invention, DPYD-1#, DPYD-2#, DPYD-3#, DPYD-4#, CD gene and shTSPO were synthesized by Hunan Yada Fenghui New Materials Co., Ltd.; and Vero cells were purchased from the ATCC under the item number of CCL-81.

### Test Example 1

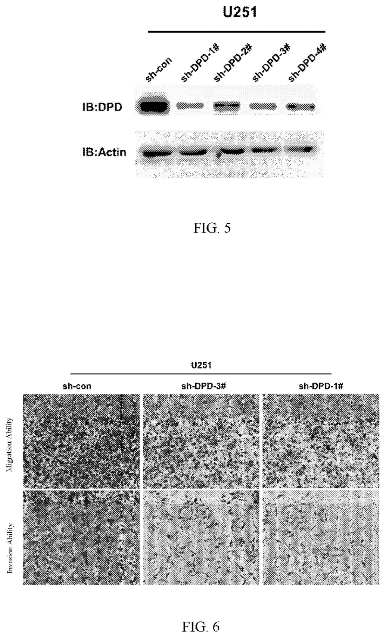

The recombinant herpes simplex viruses in the above-described Example 2 (sh-DPD-1#), Example 3 (sh-DPD-2#), Example 4 (sh-DPD-3#), Example 5 (sh-DPD-4#), and Comparative Example 1 (sh-con) were transfected into glioma cells U251, respectively, wherein the transfection process (using the Lipofectamine^{®}3000 transfection reagent, purchased from Thermo Company, and carried out according to the instructions) comprised: inoculating the glioma cells U251 into a 24-well plate, which were proliferated and cultured at 37°C and 5% CO₂; after attachment to the wall, adding the respective recombinant herpes simplex viruses constructed in Examples 2-5 and Comparative Example 1 (with MOI=0.01) to infect the glioma cells U251, respectively; collecting the cells after being cultured at 37°C, 5% CO₂ for 24 hours, detecting the expression of DPD protein in the cells transfected with the sh-DPD-1#, sh-DPD-2#, sh-DPD-3#, sh-DPD-4#, and sh-con by Western blot, to obtain the interference efficiencies of different nucleotide sequences for reducing the expression level of DPD gene; wherein nornal cells which were not transfected with the DPD-interfering shRNA *(i.e.,* sh-con) were taken as the control, and actin (Actin) was taken as the reference protein. The test results are shown in FIG. 5. It can be seen from FIG. 5 that DPYD-1#, DPYD-2#, DPYD-3# and DPYD-4 # can all effectively reduce the expression of DPD gene, wherein DPYD-1# and DPYD-3# have the best effect on reducing the expression of DPD gene, that is, they have higher efficiency of interfering with the expression of DPD gene.

### Test Example 2

The recombinant herpes simplex viruses in the above Example 2 (sh-DPD-1#), Example 4 (sh-DPD-3#) and Comparative Example 1 (sh-con) were transfected into glioma cells U251 (by the same transfection method as in Test Example 1). After the sh-DPD-1#, sh-DPD-3# and sh-con were transfected into the glioma cells U251, the cells were detected for their invasion ability and migration ability by Transwell assay. The test results were shown in FIG. 6. It can be seen from FIG. 6 that after DPYD-1# and DPYD-3# (that is, the DPD-interfering shRNAs) are expressed, the invasion and migration abilities of glioma cells are reduced, and DPYD- 1# *(i.e.,* sh-DPD-1#) is more effective in interfering with the DPD expression than DPYD-3# *(i.e.,* sh-DPD-3#). The better the effect of interfering with the DPD expression, the more obvious the effect of inhibiting cell invasion and migration.

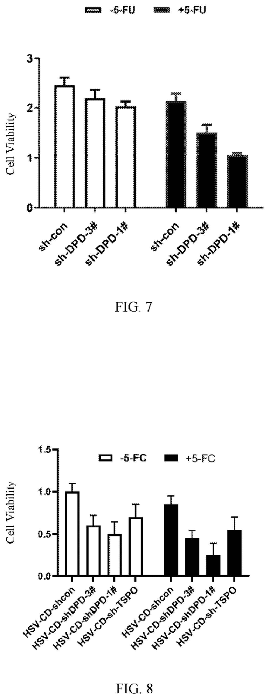

After DPYD-1# and DPYD-3# interfered with the expression of DPD, the viability of tumor cells was detected by CCK8 to detect the ability of 5-FU to kill cells. The test results are shown in FIG. 7. It can be seen from FIG. 7 that, in the case that no 5-FU is added *(i.e.,* "-5-FU" in FIG. 7), the viability of glioma cells decreases after the DPD expression is interfered; and in the case that 5-FU is added *(i.e.,* "+5-FU" in FIG. 7), the 5-FU (with the 5-FU concentration of 100 µmol/L) can effectively kill the glioma cells. The viability of glioma cells further decreases after the 5-FU is added into the cells in which the DPD expression is interfered, and the effect of DPYD-1# (ie sh-DPD-1#) is better than that of DPYD-3# (sh-DPD-3#), showing that after the DPD expression is interfered, the glioma cells are more sensitive to 5-FU, increasing the half-life of chemotherapeutic drugs and the chemotherapy efficiency in glioma cells.

### Test Example 3

The recombinant herpes simplex viruses in the above Example2 (sh-DPD-1#, *i.e.,* HSV-CD-shDPD-1#), Example 4 (sh-DPD-3#, *i.e.,* HSV-CD-shDPD-3#), and Comparative Example 2 (HSV-CD-sh-TSPO) were transfected into glioma cells U251, respectively (by the same transfection method as in Test Example 1). After the sh-DPD-1#, sh-DPD-3#, and HSV-CD-sh-TSPO were transfected into the glioma cell U251, DPYD-1# and DPYD-3# interfered with the expression of DPD, and the viability of tumor cells was detected by CCK8 to detect the ability of 5-FU to kill cells. The test results are shown in FIG. 8. It can be seen from FIG. 8 that, in the case that no 5-FC is added *(i.e.,* "-5-FU" in FIG. 8), the viability of glioma cells decreases after the DPD expression is interfered, and the viability of glioma cells in which the DPD expression is interfered is lower than the viability of glioma cells in which the TSPO is interfered; and in the case that 5-FC is added *(i.e.,* "+5-FU" in FIG. 8), the 5-FC is transformed to 5-FU via deamination catalyzed by cytosine deaminase (CD), and the 5-FU can effectively kill glioma cells. And compared with the viability of glioma cells after 5-FC is added to the cells in which the TSPO expression is interfered, the viability of glioma cells further decreases after 5-FU is added to the cells in which the DPD expression is interfered as described in the present invention, showing that inserting a nucleotide sequence for reducing the expression level of DPD gene can effectively improve the utilization rate of the inserted CD gene to the prodrug 5-FC, namely, to simultaneously improve the utilization rate of exogenous drugs.

The glioma cell line U251 in the above detection example was purchased from the ATCC under the item number TX-1725.

Although the preferred embodiments of the present invention have been disclosed above, they are not intended to limit the present invention. Any person skilled in this technology can make various variations and modifications without departing from the spirit and scope of the present invention. Therefore, the protection scope of the present invention should be defined by the claims.

## Claims

1. A recombinant herpes simplex virus, **characterized in that** the recombinant herpes simplex virus comprises a vector and a nucleotide sequence for reducing an expression level of DPD gene;
wherein the vector is a herpes simplex virus in which an ICP47-encoding gene is deleted and a CD gene is contained, and an insertion site of the nucleotide sequence for reducing the expression level of DPD gene is a site on the vector where the ICP47-encoding gene is deleted;
the nucleotide sequence for reducing the expression level of DPD gene is shown in SEQ ID NO: 1, SEQ ID NO: 2, SEQ ID NO: 3, or SEQ ID NO: 4.

2. The recombinant herpes simplex virus according to claim 1, **characterized in that** an insertion site of the CD gene in the vector is a site where the ICP34.5-encoding gene is deleted.

3. The recombinant herpes simplex virus according to claim 1, **characterized in that** the DPD gene has a nucleotide sequence as shown in SEQ ID NO: 5.

4. The recombinant herpes simplex virus according to claim 1, **characterized in that** the CD gene has a nucleotide sequence as shown in SEQ ID NO: 6.

5. The recombinant herpes simplex virus according to claim 1, **characterized in that** the vector is obtained by means of:
knocking out an ICP34.5-encoding gene in the herpes simplex virus, and inserting the CD gene into a site where the ICP34.5-encoding gene is knocked out, to afford a CD gene-containing herpes simplex virus; and
knocking out the ICP47-encoding gene in the CD gene-containing herpes simplex virus, to afford a vector.

6. A method for preparing the recombinant herpes simplex virus according to any one of claims 1 to 5, **characterized by** comprising steps of:
knocking out the ICP47-encoding gene in the CD gene-containing herpes simplex virus to afford a vector; and inserting the nucleotide sequence for reducing the expression level of DPD gene into the vector; wherein an insertion site of the nucleotide sequence for reducing the expression level of DPD gene is a site on the vector where the ICP47-encoding gene is knocked out; and
wherein the nucleotide sequence for reducing the expression level of DPD gene is shown in SEQ ID NO: 1, SEQ ID NO: 2, SEQ ID NO: 3, or SEQ ID NO: 4.

7. The method for preparing the recombinant herpes simplex virus according to claim 6, **characterized in that** the CD gene-containing herpes simplex virus is obtained by means of:
knocking out the ICP34.5-encoding gene in the herpes simplex virus, and inserting the CD gene into a site where the ICP34.5-encoding gene is knocked out, to afford the CD gene-containing herpes simplex virus.

8. The method for preparing the recombinant herpes simplex virus according to claim 7, **characterized by** further comprising the steps of:
synthesizing the CD gene, and cloning the CD gene into the pSP72ΔICP34.5CMVWPRE vector using Hind III and Xho I to afford pSP72ΔICP34.5CMVCDWPRE; co-transfecting BHK cells with pSP72ΔICP34.5CMVCDWPRE and HSV-1ΔICP34.5CMVEGFP, wherein virus spots that do not express a green fluorescence indicate that the gene EGFP in the original virus is replaced by CD gene recombination; purifying and growing an HSV-1ΔICP34.5CMVCDWPRE virus vector, and extracting a full-length DNA of the vector, to afford the CD gene-containing herpes simplex virus.

9. Use of the recombinant herpes simplex virus according to any one of claims 1 to 5 or the recombinant herpes simplex virus prepared by the method according to any one of claims 6 to 8 in preparation of a drug for treating glioma.
